# FASCICULE DE BREVET EUROPEEN

(11) **EP 1 043 346 B1**
(45) Date de publication et mention de la délivrance du brevet: **05.03.2003**
(21) Numéro de dépôt: 00400651.6
(22) Date de dépôt: 09.03.2000
(51) Int. Cl.: C08F 293/00, C08G 81/02, A61K 7/06

(54) **Composition cosmétique comprenant des polymères ayant une structure en étoiles et leur utilisation notamment en capillaire**
Kosmetische Zusammensetzungen aus sternförmigen Polymeren und ihre Anwendung insbesondere für Haare
Cosmetic compositions containing star shaped polymers and their use in particular for hair

(30) Priorité: 06.04.1999 FR 9904258
(43) Date de publication de la demande: 11.10.2000
(73) Titulaire: L'OREAL, 75008 Paris (FR)
(72) Inventeur: Mougin, Nathalie, 75011 Paris (FR)
(74) Mandataire: Dodin, Catherine

(56) Documents cités:
- WO-A-86/00626
- DE-A- 4 328 004
- DE-A- 19 602 540
- US-A- 3 907 984
- US-A- 5 804 664

## Description

L'invention a pour objet une composition susceptible d'être appliquée sur les cheveux, notamment une composition de coiffage comprenant, dans un milieu cosmétiquement acceptable, au moins un polymère de structure ordonnée bien particulière. Elle vise également un procédé de mise en forme ou de maintien des cheveux à l'aide de cette composition ainsi que son utilisation pour la formulation de produits de coiffage tels que les laques, les sprays ou les mousses, en vue d'obtenir le maintien ou la mise en forme de la coiffure.

Les produits capillaires pour la fixation des cheveux les plus répandus sur le marché de la cosmétique sont des compositions à pulvériser en aérosol ou en flacon-pompe telles que les laques, les sprays ou les mousses, essentiellement constituées d'une solution le plus souvent alcoolique ou hydroalcoolique et d'un polymère filmogène soluble dans l'eau ou dans l'alcool, en mélange avec divers adjuvants cosmétiques.
Toutefois, ces formulations capillaires telles que les mousses, les gels et surtout les sprays et les laques aérosols destinées à maintenir la forme de la coiffure, ne permettent pas à la coiffure de résister de manière satisfaisante aux différents mouvements naturels de la vie comme la marche, les mouvements de tête ou les coups de vent. En effet, les polymères utilisés pour la formulation de ces produits capillaires sont généralement des polymères filmogènes anioniques, amphotères ou non ioniques, qui conduisent à la formation de films possédant un caractère plus ou moins dur et cassant. Lorsque le polymère est trop cassant, le pourcentage d'allongement à la rupture mesuré sur le film est faible, c'est-à-dire en général inférieur à 2 % et la tenue de la coiffure n'est pas assurée dans le temps.

Pour remédier à ce problème, on a déjà mélangé ces polymères avec des plastifiants et obtenu des revêtements plus souples et non friables. Toutefois, ces films sont déformables et plastiques, c'est-à-dire qu'après déformation, ils ne récupèrent que très peu de leur forme initiale. Si la tenue de la coiffure est améliorée, elle n'est pas encore satisfaisante puisque la forme de la coiffure évolue dans le temps.

Des résultats plus satisfaisants en terme de tenue ont été obtenus avec des compositions comprenant une association de polymères filmogènes, tels que par exemple un polymère cellulosique et un polymère acrylique. Toutefois, ces compositions ne donnent encore pas entièrement satisfaction, dans la mesure où les cheveux perdent certaines de leurs propriétés cosmétiques naturelles.

Par ailleurs, on connaît, par le document WO86/006 des polymères étoiles de type acryliques, susceptibles d'être employés notamment comme agent renforçateur dans les domaines des matériaux composites, des matériaux plastiques, des revêtements pour automobiles ou camions.

On connaît également par le document US5804664 des polymères étoiles à branches multiples, comprenant au moins un polyisobutylène, et pouvant être employés comme agent modificateur de viscosité ou comme additif pour les huiles de moteur.
On connaît encore par DE4328004 des copolymères blocs, notamment diblocs, à base de polyméthacrylates et/ou de polyacrylates, qui trouvent une application dans le domaine thermoplastique.

On recherche donc des compositions cosmétiques pour le maintien et/ou la fixation de la coiffure qui procurent à la chevelure, outre une fixation durable, de bonnes propriétés cosmétiques, notamment un bon démêlage, de la douceur et un aspect agréable.

La demanderesse a constaté que, de façon surprenante et inattendue, l'utilisation de polymères bien particuliers, présentant une structure ordonnée spécifique, pouvait permettre l'obtention d'une composition susceptible d'être appliquée sur les fibres kératiniques, notamment les cheveux, et qui pouvait permettre un maintien adéquat de la chevelure, tout en leur conservant un démêlage facile et un aspect agréable.

Ainsi, un objet de la présent invention est une composition cosmétique capillaire comprenant, dans un milieu cosmétiquement acceptable défini, au moins un polymère de structure "en étoiles" tel que défini ci-après et dans les revendications.

Un autre objet de l'invention est un procédé de maintien ou de mise en forme des cheveux, caractérisé par le fait qu'il consiste à appliquer sur ceux-ci une composition cosmétique telle que ci-dessus définie.

Un autre objet de l'invention est l'utilisation d'une composition cosmétique telle que ci-dessus définie pour la fabrication d'un produit cosmétique capillaire destiné à maintenir et/ou fixer et/ou traiter la coiffure et/ou les cheveux.

La composition selon l'invention comprend donc un polymère dont la structure en "étoiles" peut être illustrée, de manière générale, par la formule suivante (I) :

A-[(M1)ₚ₁ - (M2)ₚ₂ .... (Mi)ₚⱼ]ₙ

dans laquelle
- A représente un centre multifonctionnel, de fonctionnalité "n", n étant un entier supérieur, de préférence compris entre 4 et 10,
- [(M1)ₚ₁ - (M2)ₚ₂ .... (Mi)ₚⱼ] représente une chaîne polymérique, aussi appelée "branche", constituée de monomères Mi polymérisés, identiques ou différents, ayant un indice de polymérisation pj, chaque branche étant identique ou différente, et étant greffée de manière covalente sur ledit centre A,
- i étant supérieur ou égal à 2, de préférence compris entre 2 et 10;
- pj étant supérieur ou égal à 2, de préférence compris entre 10 et 20 000.

De préférence, les chaînes polymériques se présentent sous forme de blocs, de masse moléculaire supérieure ou égale à 500, pouvant aller jusqu'à 2 000 000.

Le polymère utilisé dans le cadre de la présente invention peut être obtenu par polymérisation radicalaire contrôlée, également appelée polymérisation radicalaire "vivante". Cette technique permet notamment de surmonter les limitations inhérentes à la polymérisation radicalaire classique, c'est-à-dire qu'elle permet notamment de contrôler la longueur des chaînes du polymère formé, et de ce fait d'obtenir des structures blocs.
La polymérisation radicalaire contrôlée permet de réduire les réactions de désactivation de l'espèce radicalaire en croissance, en particulier l'étape de terminaison, réactions qui, dans la polymérisation radicalaire classique, interrompent la croissance de la chaîne polymérique, de façon irréversible et sans contrôle.
Afin de diminuer la probabilité des réactions de terminaison, il a été proposé de bloquer, de façon transitoire et réversible, l'espèce radicalaire en croissance, en formant des espèces actives dites "dormantes" à l'aide de liaison de faible énergie de dissociation.

Des liaisons de type C―halogénure (en présence de complexe métal/ligand) sont utilisées. On parle alors de polymérisation radicalaire par transfert d'atomes, aussi connue sous l'abréviation ATRP.

Ce type de polymérisation se traduit par un contrôle de la masse des polymères formés et par un faible indice de polydispersité en poids des chaînes.
D'une manière générale, la polymérisation radicalaire par transfert d'atomes s'effectue par polymérisation :
- d'un ou plusieurs monomères polymérisables radicalairement, en présence
- d'un initiateur ayant au moins un atome ou un groupe radicalairement transférable,
- d'un composé comprenant un métal de transition, susceptible de participer à une étape de réduction avec l'initiateur et une chaîne polymérique "dormante", et
- d'un ligand, pouvant être choisi parmi les composés comprenant un atome d'azote (N), d'oxygène (O), de phosphore (P) ou de soufre (S), susceptibles de se coordonner par une liaison σ audit composé comprenant un métal de transition, ou parmi les composés comprenant un atome de carbone susceptibles de se coordonner par une liaison π ou σ audit composé comprenant un métal de transition, la formation de liaisons directes entre ledit composé comprenant un métal de transition et le polymère en formation étant évitées.
Ce procédé est en particulier illustré dans la demande WO97/18247, dont l'homme du métier pourra tirer enseignement pour préparer les polymères entrant dans le cadre de la présente invention.

La nature et la quantité des monomères, initiateur(s), composé(s) comprenant le métal de transition et ligand(s) seront choisies par l'homme du métier sur la base de ses connaissances générales, en fonction du résultat recherché.

En particulier, les monomères "M" peuvent être choisis, seuls ou en mélange, parmi les composés à insaturation éthylénique, radicalairement polymérisables, répondant à la formule : 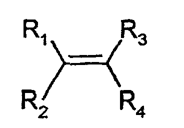 dans laquelle R₁, R₂, R₃ et R₄ sont, indépendamment les uns des autres, choisis parmi:
- un atome d'hydrogène;
- un atome d'halogène;
- un radical alkyle, linéaire ou ramifié, ayant 1 à 20, de préférence 1-6, plus préférentiellement 1-4, atomes de carbone, éventuellement substitué par un ou plusieurs halogènes et/ou un ou plusieurs radicaux -OH;
- un radical alcényle ou alkynyle, linéaire ou ramifié, ayant 2 à 10, de préférence 2-6, plus préférentiellement 2-4, atomes de carbone, éventuellement substitué par un ou plusieurs halogènes;
- un radical hydrocarboné cyclique (cycloalkyle) ayant 3 à 8 atomes de carbone, éventuellement substitué par un ou plusieurs atomes d'halogène, d'azote, de soufre, d'oxygène;
- un radical choisi parmi CN, C(=Y)R⁵, C(=Y)NR⁶R⁷, YC(=Y)R⁵, NC(=Y)R⁵ cyclique, SOR⁵, SO₂R⁵, OSO₂R⁵, NR⁸SO₂R⁵, PR⁵₂, P(=Y)R⁵₂, YPR⁵₂, YP(=Y)R⁵₂, NR⁸₂ qui peut être quaternisé avec un groupe additionnel R⁸, aryle et hétérocyclyle. avec :
   - Y représente O, S ou NR⁸ (de préférence O),
   - R⁵ représente un radical alkyle, alkylthio, alcoxy, linéaire ou ramifié, ayant 1-20 atomes de carbone; un radical OH; un radical OM' avec M' = métal alcalin; un radical aryloxy ou un radical heterocyclyloxy;
   - R⁶ et R⁷ représentent, indépendamment l'un de l'autre, H ou un radical alkyle, linéaire ou ramifié, ayant 1-20 atomes de carbone; étant donné que R⁶ et R⁷ peuvent être joints pour former un groupe alkylène ayant 2-7, de préférence 2-5, atomes de carbone;
   - R⁸ représente H; un radical alkyle, linéaire ou ramifié, ayant 1-20 atomes de carbone ou un radical aryle;
- un radical -COOR dans lequel R est un radical alkyle, linéaire ou ramifié, ayant 1 à 20, de préférence 1-6, atomes de carbone, éventuellement substitué par un ou plusieurs halogènes;
- un radical -CONHR' dans lequel R' est l'hydrogène ou un radical hydrocarboné, saturé ou insaturé, linéaire ou ramifié, ayant 1 à 20, de préférence 1-6, atomes de carbone, éventuellement substitué par un ou plusieurs halogènes, azotes et/ou oxygènes;
- un radical -OCOR" dans lequel R" est l'hydrogène ou un radical hydrocarboné, saturé ou insaturé, linéaire ou ramifié, ayant 1 à 20 atomes de carbone, éventuellement substitué par un ou plusieurs halogènes, azotes et/ou oxygènes;
- un radical comprenant au moins un atome de silicium, et notamment des radicaux tels que : un radical -R-siloxane; un radical -CONHR-siloxane; un radical -COOR-siloxane ou un radical -OCO-R-siloxane, dans lesquels R est un radical alkyle, alkylthio, alcoxy, aryloxy ou hétérocycloxy, linéaire ou ramifié, ayant 1-20 atomes de carbone.

Par siloxane, on entend un composé comprenant des motifs (-SiR^{a}R^{b}O-)n, dans lesquels R^{a} et R^{b} peuvent représenter, indépendamment l'un de l'autre, un hydrogène; un halogène; un radical hydrocarboné, linéaire ou ramifié, saturé ou insaturé, ayant 1 à 36 atomes de carbone, éventuellement substitué par un ou plusieurs halogènes, azotes et/ou oxygènes; un radical hydrocarboné cyclique ayant 1 à 20 atomes de carbone; n étant supérieur ou égal à 1.

Notamment on peut citer les polydiméthylsiloxanes (PDMS) comprenant 1 à 200, de préférence moins de 100, motifs de répétition.

Par ailleurs, R¹ et R³ peuvent être reliés entre eux de manière à former un cycle de formule (CH₂)n, qui peut être substitué par un ou plusieurs halogènes et/ou oxygènes et/ou azote, et/ou par des radicaux alkyles ayant 1 à 6 atomes de carbone.

Par 'aryle' ou 'hétérocyclyle' on entend la définition communément comprise par l'homme du métier et qui peut être illustrée par l'art antérieur WO97/18247.

De préférence, on peut choisir les monomères M dans le groupe constitué par :
- les esters acryliques ou méthacryliques obtenus à partir d'alcools aliphatiques, linéaires, ramifiés, cycliques et/ou d'alcools aromatiques, de préférence en C₁-C₂₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle ;
- les (méth)acrylates d'hydroxyalkyle en C₁-C₄ tels que le (méth)acrylate de 2-hydroxyéthyle ou le (méth)acrylate de 2-hydroxypropyle;
- les (méth)acrylates d'éthylèneglycol, de diéthylèneglycol, de polyéthylèneglycol, à extrémité hydroxyle ou éther;
- les esters vinyliques, allyliques ou méthallyliques obtenus à partir d'alcools aliphatiques, linéaires ou ramifiés en C₁-C₁₀ ou cycliques en C₁-C₆ et/ou d'alcools aromatiques, de préférence en C₁-C₆, tels que l'acétate de vinyle, le propionate de vinyle, le benzoate de vinyle, le tertio-butyl benzoate de vinyle ;
- la N-vinylpyrrolidone; la vinylcaprolactame; les vinyl N-alkylpyrroles ayant 1 à 6 atomes de carbone; les vinyl-oxazoles; les vinyl-thiazoles; les vinylpyrrimidines; les vinylimidazoles; les vinyl cétones;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les oléfines tels que l'éthylène, le propylène, le styrène ou le styrène substitué;
- les monomères acryliques ou vinyliques fluorés ou perfluorés, notamment les esters (méth)acryliques à motifs perfluoroalkyle;
- les monomères comportant une fonction amine sous forme libre ou bien partiellement ou totalement neutralisée ou bien partiellement ou totalement quatemisée tels que le (méth)acrylate de diméthylaminoéthyle, le diméthylaminoéthyl méthacrylamide, la vinylamine, la vinylpyridine, le chlorure de diallyldiméthylammonium;
- les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium par exemple) ou par des sulfones cycliques (propane sulfone);
- les (méth)acrylates ou (méth)acrylamides siliconés, notamment les esters(méth)acryliques à motifs siloxané;
- leurs mélanges.

Les monomères particulièrement préférés sont choisis parmi :
- les esters (méth)acryliques obtenus à partir d'alcools aliphatiques, linéaires ou ramifiés, de préférence en C₁-C₂₀;
- les esters (méth)acryliques en C₁-C₂₀ à motifs perfluoroalkyle;
- les esters(méth)acryliques en C₁-C₂₀ motifs siloxane;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les esters vinyliques, allyliques ou méthallyliques obtenus à partir d'alcools aliphatiques, linéaires ou ramifiés en C₁-C₁₀ ou cycliques en C₁-C₆ ;
- la vinylcaprolactame ;
- le styrène éventuellement substitué;
- leurs mélanges.

Dans le cadre de la présente invention, l'initiateur peut être tout composé, notamment moléculaire ou polymérique, ayant au moins deux atomes et/ou groupes radicalairement transférables par polymérisation.
Notamment, l'initiateur peut être un oligomère ou un polymère susceptible d'être obtenu par polymérisation radicalaire, par polycondensation, par polymérisation anionique ou cationique, ou par ouverture de cycles.
Lesdits atomes et/ou groupes transférables peuvent être situés aux extrémités de la chaîne polymérique ou le long du squelette.

En particulier, on peut citer les composés correspondant à l'une des formules suivantes :
- R¹¹ₓ R¹²_{y} R¹³_{z} C-(RX)ₜ dans laquelle x, y et z représentent un entier allant de 0 à 4, t un entier allant de 1 à 4, et x+y+z= 4-t;
- R¹³ₓ C₆-(RX)_{y} (cycle à 6 carbones, saturé) dans laquelle x représente un entier allant de 7 à 11, y représente un entier allant de 1 à 5, et x+y= 12 ;
- R¹³ₓ C₆ -(RX)_{y} (cycle à 6 carbones, insaturé) dans laquelle x représente un entier allant de 0 à 5, y représente un entier allant de 1 à 6, et x+y= 6;
- -[-(R¹¹)(R¹²)(R¹³)C-(RX)-]ₙ dans laquelle n est supérieur ou égal à 1; cyclique ou linéaire;
- -[-(R¹²)ₓC₆(RX)_{y}-R¹¹-]ₙ dans laquelle x représente un entier allant de 0 à 6, y représente un entier allant de 1 à 6, et n est supérieur ou égal à 1, avec x+y=4 ou 6; cyclique ou linéaire;
- -[-(R¹²)ₓC₆(RX)_{y}-R¹¹-]ₙ dans laquelle x représente un entier allant de 0 à 12, y représente un entier allant de 1 à 12, et n est supérieur ou égal à 1, avec x+y=10 ou 12; cyclique ou linéaire;
- -[OSi(R¹¹)ₓ(RX)_{y}]ₙ, cyclique ou linéaire, dans laquelle x et y représentent un entier allant de 0 à 2, et n est supérieur ou égal à 1, avec x+y=2;
- R¹¹N-X₂
- (R¹¹)ₓP(O)_{y}-X₃₋ₓ dans laquelle x et y représentent des entiers allant de 0 à 2, et x+y = 5;
- (R¹¹O)ₓP(O)_{y}-X₃₋ₓ dans laquelle x et y représentent des entiers allant de 0 à 2, et x+y = 5;
- -[(R¹¹)ₜN_{z}P(O)ₓ(O-RX)_{y}-]ₙ, cyclique ou linéaire, dans laquelle x représente un entier allant de 0 à 4, y représente un entier allant de 1 à 5, z représente un entier allant de 0 à 2 et t représente un entier allant de 0 à 3, et n est supérieur ou égal à 1;
dans lesquelles :
- R, R¹¹, R¹² et R¹³ représentent, indépendamment l'un de l'autre, un atome d'hydrogène ou d'halogène; un radical alkyle, linéaire ou ramifié, ayant 1-20, de préférence 1-10 et plus préférentiellement 1-6 atomes de carbone; un radical cycloalkyle ayant 3-8 atomes de carbone; un radical -C(=Y)R⁵, -C(=Y)NR⁶R⁷ ou -R⁸₃Si (voir les définitions de R⁵ à R⁸ ci-dessus); -COCI, -OH, -CN, un radical alkényle ou alkynyle ayant 2-20, de préférence 2-6, atomes de carbone; un radical oxiranyle, glycidyle, alkylène ou alkénylène substitué avec un oxiranyle ou un glycidyle; un radical aryle, heterocyclyle, aralkyle, aralkenyle; un radical alkyle ayant 1-6 atomes de carbone dans lequel tout ou partie des atomes d'hydrogène sont substitués soit par des atomes d'halogènes tels que fluor, chlore ou brome, soit par un groupe alcoxy ayant 1-4 atomes de carbone ou par un radical aryle, heterocyclyle, -C(=Y)R⁵, -C(=Y)NR⁶R⁷, oxiranyle, glycidyle;
- X représente un atome d'halogène tel que Cl, Br, I, ou un radical -OR', -SR, -SeR, -OC(=O)R', -OP(=O)R', -OP(=O)(OR')₂, -OP(=O)OR', -O-NR'₂, -S-C(=S)NR'₂, -CN, -NC, -SCN, -CNS, -OCN, -CNO et -N₃, dans lequel R' représente un radical alkyle ayant 1-20 atomes de carbone éventuellement substitué par un ou plusieurs atomes d'halogènes notamment de fluor et/ou de chlore; et R représente un radical aryle ou alkyle, linéaire ou ramifié, ayant 1-20, de préférence 1-10, atomes de carbone; le groupement -NR'₂ pouvant en outre représenter un groupement cyclique, les deux groupes R' étant joints de manière à former un hétérocycle à 5, 6 ou 7 membres.

De préférence, X représente un atome d'halogène et notamment un atome de chlore ou de brome.

De préférence, on choisit l'initiateur parmi les composés de formule
- R¹³ₓC₆-(RX)_{y} (cycle à 6 carbones, saturé) dans laquelle x représente un entier allant de 7 à 11, y représente un entier allant de 1 à 5, et x+y= 12 ;
- -[-(R¹²)ₓC₆(RX)_{y}-R¹¹-]ₙ dans laquelle x représente un entier allant de 0 à 6, y représente un entier allant de 1 à 6, et n est supérieur ou égal à 1, avec x+y=4 ou 6; cyclique ou linéaire; et
- -[OSi(R¹¹)ₓ(RX)_{y}]ₙ, cyclique ou linéaire, dans laquelle x et y représentent un entier allant de 0 à 2, et n est supérieur ou égal à 1, avec x+y=2.

En particulier, on peut citer comme initiateur, les composés suivants :
- l'octa-2-isobutyrylbromide-octatertiobutyl-calix(8)arène,
- l'octa-2-propionylbromide-octatertiobutyl-calix(8)arène, et
- l'hexakis α-bromométhylbenzène.

Le composé comprenant un métal de transition, susceptible de participer à une étape de réduction avec l'initiateur et une chaîne polymérique "dormante", peut être choisi parmi ceux qui correspondent à la formule Mⁿ⁺X'ₙ, dans laquelle :
- M peut être choisi parmi Cu, Au, Ag, Hg, Ni, Pd, Pt, Rh, Co, Ir, Fe, Ru, Os, Re, Mn, Cr, Mo, W, V, Nb, Ta et Zn,
- X' peut représenter un halogène (brome ou chlore notamment), OH, (O)_{1/2}, un radical alcoxy ayant 1-6 atomes de carbone, (SO₄)_{1/2}, (PO₄)_{1/3}, (HPO₄)_{1/2}, (H₂PO₄), un radical triflate, hexafluorophosphate, methanesulfonate, arylsulfonate, SeR, CN, NC, SCN, CNS, OCN, CNO, N₃ et R'CO₂, dans lequel R représente un radical aryle ou alkyle, linéaire ou ramifié, ayant 1-20, de préférence 1-10 atomes de carbone, et R' représente H ou un radical alkyle, linéaire ou ramifié, ayant 1-6 atomes de carbone, ou un radical aryle, éventuellement substitué par un ou plusieurs atomes d'halogènes notamment de fluor et/ou de chlore;
- n est la charge du métal.

De préférence, on choisit M représentant le cuivre ou le ruthénium, et X' représentant le brome ou le chlore.
En particulier, on peut citer le bromure de cuivre.

Parmi les ligands susceptibles d'être utilisés dans le cadre de la présente invention, on peut citer les composés comprenant au moins un atome d'azote, d'oxygène, de phosphore et/ou de soufre, susceptibles de se coordonner par une liaison σ au composé comprenant un métal de transition.
On peut aussi citer les composés comprenant au moins deux atomes de carbone susceptibles de se coordonner par une liaison π audit composé comprenant un métal de transition.
On peut encore citer les composés comprenant au moins un atome de carbone susceptibles de se coordonner par une liaison σ audit composé comprenant un métal de transition, mais qui ne forment pas de liaison carbone-carbone avec le monomère lors de la polymérisation, c'est-à-dire qui ne participent pas à des réactions de β-addition avec les monomères.
On peut encore citer les composés susceptibles de se coordonner par une liaison µ ou η audit composé comprenant un métal de transition.

Notamment, on peut citer les composés de formule : R⁹-Z-(R¹⁴-Z)ₘ-R¹⁰
dans laquelle :
- R⁹ et R¹⁰ sont, indépendamment l'un de l'autre, un atome d'hydrogène; un radical alkyle, linéaire ou ramifié, ayant 1-20, de préférence 1-10 atomes de carbone; un radical aryle; un radical hétérocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸ (voir les définitions de R⁵ à R⁸ et Y ci-dessus);
   étant donné que R⁹ et R¹⁰ peuvent être joints de manière à former un cycle, saturé ou insaturé;
- R¹⁴ représente, indépendamment les uns des autres, un groupe divalent choisi parmi les alcanediyles ayant 2-4 atomes de carbone; les alkénylènes ayant 2-4 atomes de carbone; les cycloalcanediyles ayant 3-8 atomes de carbone; les cycloalkènediyles ayant 3-8 atomes de carbone; les arènediyles et les hétérocyclylènes;
- Z représente O, S, NR¹⁵ ou PR¹⁵ avec R¹⁵ représentant H; un radical alkyle, linéaire ou ramifié, ayant 1-20 atomes de carbone; un radical aryle; un radical heterocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸ (voir les définitions de R⁵ à R⁸ et Y ci-dessus);
- m est compris entre 0 et 6.

On peut également citer les composés de formule : R²⁰R²¹C[C(=Y)R⁵]
dans laquelle :
- R²⁰ et R²¹ sont, indépendamment l'un de l'autre, un atome d'hydrogène; un atome d'halogène; un radical alkyle, linéaire ou ramifié, ayant 1-20, de préférence 1-10 atomes de carbone; un radical aryle; un radical hétérocyclyle; étant donné que R²⁰ et R²¹ peuvent être joints de manière à former un cycle, saturé ou insaturé; étant donné que chaque radical peut en outre être substitué avec un radical alkyle ayant 1-6 atomes de carbone, un radical alcoxy ayant 1-6 atomes de carbone ou un radical aryle;
- R⁵ et Y étant définis ci-dessus.

On peut encore citer comme ligands, le monoxyde de carbone; les porphyrines et les porphycènes, éventuellement substitués; l'éthylènediamine et le propylènediamine, éventuellement substitués; les multiamines avec amines tertiaires telles que le pentaméthyldiéthylènetriamine; les aminoalcools tels que l'aminoéthanol et l'aminoprpoanol, éventuellement substitués; les glycols tels que l'éthylèneglycol ou le propylèneglycol, éventuellement substitués; les arènes tels que le benzène, éventuellement substitués; le cyclopentadiène, éventuellement substitué; les pyridines et bipyridines, éventuellement substituées; l'acétonitrile; la 1,10-phénanthroline; les cryptands et les éthers-couronnes; la spartéine.

Les ligands préférés sont choisis notamment parmi les pyridines et bipyridines, éventuellement substituées par des radicaux alkyls en C2-C15, en particulier en C6-C12, et notamment le radical nonyle; les multiamines avec amines tertiaires telles que la pentaméthyldiéthylènetriamine.

La polymérisation des monomères, en présence de l'initiateur, du composé comprenant un métal de transition et du ligand qui joue le rôle d'activateur, conduit à l'obtention d'un polymère ayant une structure d'étoiles, qui peut être représentée par la formule (I) donnée ci-dessus, dans laquelle les monomères se sont polymérisés pour donner "n" chaînes polymériques, semblables ou différentes, toutes reliées à un centre multifonctionnel A qui dérive de l'initiateur.

On a constaté que pour atteindre le but poursuivi par la présente invention, c'est-à-dire obtenir une composition qui ne présente pas les inconvénients de l'art antérieur et qui permette en particulier l'obtention d'une bonne fixation tout en conservant un démêlage aisé de la chevelure, il était nécessaire de choisir un polymère répondant aux critères suivants :
- il doit comprendre un ou plusieurs monomères Mi dont l'homopolymère correspondant présente une Tg supérieure ou égale à environ 10°C, de préférence supérieure ou égale à 15°C, et encore mieux supérieure ou égale à 20°C;
- ce ou ces monomères Mi étant présents, dans le polymère final, en une quantité comprise entre 55 et 95% en poids, de préférence en une quantité de 60 à 93% en poids, et encore mieux en une quantité de 65-90% en poids, par rapport au poids total de monomères;
et
- il doit comprendre par ailleurs, un ou plusieurs monomères Mj dont l'homopolymère correspondant présente une Tg inférieure ou égale à environ 10°C, de préférence inférieure ou égale à 5°C, et encore mieux inférieure ou égale à 0°C;
- ce ou ces monomères Mj étant présents, dans le polymère final, en une quantité comprise entre 5 et 45% en poids, de préférence en une quantité de 7 à 40% en poids, et encore mieux en une quantité de 10-35% en poids, par rapport au poids total de monomères.

La mesure de Tg (température de transition vitreuse) est effectuée par DSC (Differential Scanning Calorimetry) selon la norme ASTM D3418-97.

Les polymères tels que définis dans la présente invention doivent être filmogènes ou peuvent être rendus filmogènes par addition d'un agent auxiliaire de filmification. Par filmogène, on entend que le polymère, après application sur un support et évaporation du solvant (aqueux ou organique) conduit à un film transparent et non craquelé.
Un tel agent auxiliaire de filmification peut être choisi parmi tous les composés connus de l'homme du métier comme étant susceptibles de remplir la fonction recherchée, et peut être notamment choisi parmi les agents plastifiants et/ou parmi les agents de coalescence. En particulier, on peut citer, seuls ou en mélange :
- les glycols et leurs dérivés tels que le diéthylène glycol éthyléther, le diéthylène glycol méthyléther, le diéthylène glycol butyléther, le diéthylène glycol hexyléther, l'éthylène glycol éthyléther, l'éthylène glycol butyléther, l'éthylène glycol hexyléther;
- les esters de glycérol, tels que le diacétate de glycérol (diacétine) et le triacétate de glycérol (triacétine);
- les dérivés de propylène glycol et en particulier le propylène glycol phényléther, le propylène glycol diacétate, le propylène glycol méthyléther, le propylène glycol éthyléther, le propylène glycol butyléther, le dipropylène glycol méthyléther, le dipropylène glycol butyléther, le dipropylène glycol éthyléther, le tripropylène glycol butyléther, le tripropylène glycol méthyléther;
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone oxyéthylénées.
La quantité d'agent auxiliaire de filmification peut être choisie par l'homme du métier sur base de ses connaissances générales, de manière à un film ayant les propriétés mécaniques souhaitées, tout en conservant à la composition des propriétés cosmétiquement acceptables.

Les polymères tels que ci-dessus définis peuvent être présents dans le milieu sous forme dissoute ou en dispersion, dans une phase aqueuse, organique ou hydroorganique notamment alcoolique ou hydroalcoolique.
Lesdits polymères peuvent être présents dans les compositions selon l'invention en une quantité aisément déterminable par l'homme du métier selon l'application envisagée, et qui peut être comprise entre 1-95% en poids de matière sèche, par rapport au poids total de la composition, de préférence entre 1-50% en poids et préférentiellement entre 1-20% en poids.

Les compositions notamment cosmétiques selon l'invention comprennent donc en outre un milieu cosmétiquement acceptable, qui peut être choisi par l'homme du métier selon l'application envisagée.

Ce milieu peut comprendre une phase aqueuse et/ou une phase grasse. Il est, de préférence, constitué par de l'eau ou un ou plusieurs solvants cosmétiquement acceptables tels que des alcools ou des mélanges eau-solvant(s).
La phase aqueuse peut comprendre de l'eau et/ou une eau thermale et/ou une eau de source et/ou une eau minérale et/ou une eau florale.
Parmi les solvants organiques acceptables, on peut citer :
- les alcools en C₁-C₄ tels que l'éthanol, l'isopropanol, le n-propanol ;
- les éthers tels que le diméthoxyéthane ;
- les cétones telles que l'acétone, la méthyléthylcétone ;
- les esters d'acide carboxylique inférieurs en C₁-C₃ tels que l'acétate de méthyle, l'acétate d'éthyle.

La phase grasse peut comprendre des huiles, volatiles ou non, des gommes et/ou des cires usuelles, d'origine animale, végétale, minérale ou synthétique, seules ou en mélanges, et notamment :
- des huiles de silicone, volatiles ou non, linéaires, ramifiées ou cycliques, éventuellement organomodifiées; des silicones phénylées; des résines et des gommes de silicone liquides à température ambiante;
- des huiles minérales telles que l'huile de paraffine et de vaseline,
- des huiles d'origine animale telles que le perhydrosqualène, la lanoline;
- des huiles d'origine végétale telles que les triglycérides liquides, par exemple les huiles de tournesol, de maïs, de soja, de jojoba, de courge, de pépins de raisin, de sésame, de noisette, d'abricot, de macadamia, d'avocat, d'amande douce, de ricin, les triglycérides des acides caprylique/caprique; l'huile d'olive, l'huile d'arachide, l'huile de colza, l'huile de coprah;
- des huiles de synthèse telles que l'huile de purcellin, les isoparaffines; les alcools gras; les esters d'acides gras;
- des huiles fluorées et perfluorées; des huiles de silicones fluorées;
- des cires choisies parmi les cires animales, fossiles, végétales, minérales ou de synthèse connues, telles que les cires de paraffine, les cires de polyéthylène, les cires de Carnauba, de Candellila; les cires d'abeilles; la cire de lanoline, les cires d'insectes de Chine, la cire de riz, la cire d'Ouricurry, la cire d'Alfa, la cire de fibres de liège, la cire de canne à sucre, la cire du Japon, la cire de sumac, la cire de montan, les cires microcristallines, l'ozokérite, les cires obtenues par la synthèse de Fisher-Tropsch; les cires de silicone; leurs mélanges.

Par ailleurs, la composition selon l'invention peut contenir des adjuvants couramment utilisés dans les compositions cosmétiques, en particulier capillaires, tels que des actifs cosmétiques; des adoucissants, des antioxydants, des opacifiants, des émollients, des agents anti-mousse, des hydratants, des vitamines, des parfums, des conservateurs, des séquestrants, des filtres UV, des céramides; des antipelliculaires; des complexants; des agents anti-chute des cheveux; des agents antifongiques ou antiseptiques; des colorants; des charges et des pigments; des épaississants; des polymères fixants ou non fixants, ou conditionneurs; des agents propulseurs, des agents alcalinisants ou acidifiants; des protéines; des polymères hydrophiles; des polymères filmogènes notamment en dispersion aqueuse; des tensioactifs, notamment anioniques ou non ioniques, éventuellement siliconés.
Bien entendu, l'homme du métier veillera à choisir ce ou ces éventuels adjuvants, et/ou leur quantité, de manière telle que les propriétés avantageuses de la composition selon l'invention ne soient pas, ou substantiellement pas, altérées par l'adjonction envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.
Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.
Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.
Les compositions conformes à l'invention peuvent être appliquées sur des cheveux secs ou humides.

Les compositions selon l'invention trouvent donc une application toute particulière comme composition pour le traitement ou la fixation des cheveux.

L'invention est illustrée plus en détail dans les exemples suivants.

### Exemple 1 : Préparation de l'initiateur

L'initiateur préparé est le 5,11,17,23,29,35,41,47-octa-2-propionylbromide-49,50,51,52,53,54,55,56-octatertiobutyl-calix(8)arène (M = 2378 g).

Les réactifs utilisés sont les suivants :
- 4-tertiobutyl-calix(8)arène (8)arène (M = 1298g)
   comportant 8 motifs phénols (Aldrich) 15 g
- 2-bromopropionylbromide de formule CH₃-CHBr-COBr 59,9 g
- triéthylamine 28 g
- tétrahydrofurane (THF) 120 g

Dans un ballon muni d'agitation et d'un thermomètre, on ajoute le 4-tbutyl-calix(8)arène et le solvant THF; on laisse sous agitation pendant 10 minutes à température ambiante.
On ajoute ensuite la triéthylamine, ce qui prend environ 15 minutes.
On ajoute alors le 2-bromopropionylbromide préalablement dissous dans le THF, à une température de 5°C environ, ce qui prend 1h30 environ.
On laisse sous agitation durant 12 heures au moins, à 5°C, puis on laisse progressivement remonter la température jusqu'à température ambiante.
On concentre la solution obtenue par évaporation du THF. On précipite dans un mélange eau/glace, puis on extrait à l'éther éthylique et on sèche sur sulfate de magnésium.
On concentre la solution obtenue et on précipite dans un mélange méthanol/glace (90/10) dans un rapport composé/précipitant de 1/5.

On obtient 23 g de composé, se présentant sous forme de poudre, soit un rendement de 85%.

La caractérisation est effectuée par RMN/ GPC ou HPLC. Le composé obtenu présente des valeurs conformes à celles attendues.

### Exemple 2 : Préparation d'un polymère-étoile à 8 branches dont chaque branche est un copolymère bloc

### 1/Première étape : préparation d'un polymère-étoile à 8 branches de polyacrylate de tertiobutyle

Les réactifs employés sont les suivants:
- monomère 1: acrylate de tertiobutyle (Tg = 50°C) 115 g
- monomère 2 : acrylate de butyle (Tg = -50°C) 5 g
- initiateur (préparé selon l'exemple 1)
   (correspondant à 4.10⁻³ mole de RBr) 1,19 g
- CuBr (correspondant à 4.10⁻³ mol) 0, 57 g
- Bipyridine (correspondant à 8.10⁻³ mol) 1,25 g

Les monomères sont préalablement distillés.
Dans un réacteur hermétique, flambé et comportant une arrivée d'azote, on mélange les réactifs sauf les monomères, puis on ajoute le monomère 1.
On chauffe, sous azote, à 120°C environ puis on laisse réagir à 120°C pendant 4 heures, en coupant l'arrivée d'azote.

### 2/ Deuxième étape : formation du deuxième bloc à l'extrémité de chaque branche

On ajoute alors le monomère 2 et on laisse à nouveau réagir à 120°C, pendant 4 heures.

Après réaction, on laisse refroidir le mélange réactionnel; on obtient une solution visqueuse verte que l'on dissout dans le dichlorométhane. On passe la solution de polymère sur alumine neutre et on précipite la solution limpide obtenue dans un mélange méthanol/eau (80/20) dans un rapport polymère/précipitant de 1/5.

On obtient 115 g de polymère se présentant sous forme de produit visqueux, soit un rendement de 96%.
Ce polymère est un polymère-étoiles à 8 branches de polyacrylate d'isobutyle, dont chaque branche est un copolymère bloc : le calix(polyacrylate de tertiobutyle-bloc-polyacrylate de butyle).

La caractérisation est effectuée par GPC : THF équivalent polystyrène linéaire, détection diffusion de lumière : 350 000 g/mol (masse théorique : 240 000 environ); indice de polydispersité : 1,6.
Le polymère obtenu présente des valeurs conformes à celles attendues.
Le polymère est soluble dans l'éthanol.

### Exemple 3 : Composition de coiffage

On prépare une composition de coiffage avant conditionnement, comprenant :
- polymère de l'exemple 1 7 g
- éthanol qsp 100 g

On prépare ensuite une laque aérosol comprenant :
- composition ci-dessus 70 g
- DME 30 g

Après application de la laque sur les cheveux, on obtient un bon pouvoir coiffant, ainsi qu'un temps de séchage rapide et de bonnes propriétés cosmétique, notamment au niveau du démêlage et du toucher.

## Revendications

1. Composition cosmétique capillaire notamment pour le traitement et/ou la fixation des cheveux comprenant, dans un milieu cosmétiquement acceptable comprenant au moins un constituant choisi parmi l'eau; un ou plusieurs solvants cosmétiquement acceptables tels que les alcools en C₁-C₄, les éthers, les cétones, les esters d'acide carboxylique inférieurs en C₁-C₃ ; des actifs cosmétiques; des adoucissants, des antioxydants, des opacifiants, des agents anti-mousse, des conservateurs, des séquestrants, des filtres UV, des céramides; des antipelliculaires; des complexants; des agents anti-chute des cheveux; des agents antifongiques ou antiseptiques; des épaississants; des polymères fixants ou non fixants, ou conditionneurs; des agents propulseurs, des agents alcalinisants ou acidifiants; des polymères hydrophiles; des polymères filmogènes notamment en dispersion aqueuse; des tensioactifs, notamment anioniques ou non ioniques, éventuellement siliconés; un agent auxiliaire de filmification, tel qu'un agent plastifiant et/ou un agent de coalescence;
au moins un polymère de structure en "étoiles" susceptible d'être obtenu par polymérisation radicalaire par transfert d'atomes :
- d'un ou plusieurs monomères polymérisables radicalairement, choisis parmi :
(i) les esters acryliques ou méthacryliques obtenus à partir d'alcools aliphatiques, linéaires, ramifiés, cycliques et/ou d'alcools aromatiques, en C₁-C₂₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle ;
(ii) les (méth)acrylates d'hydroxyalkyle en C₁-C₄ tels que le (méth)acrylate de 2-hydroxyéthyle ou le (méth)acrylate de 2-hydroxypropyle;
(iii) les (méth)acrylates d'éthylèneglycol, de diéthylèneglycol, de polyéthylèneglycol, à extrémité hydroxyle ou éther;
(iv) les esters vinyliques, allyliques ou méthallyliques obtenus à partir d'alcools aliphatiques, linéaires ou ramifiés en C₁-C₁₀ ou cycliques en C₁-C₆ et/ou d'alcools aromatiques, en C₁-C₆, tels que l'acétate de vinyle, le propionate de vinyle, le benzoate de vinyle, le tertio-butyl benzoate de vinyle ;
(v) la N-vinylpyrrolidone; la vinylcaprolactame; les vinyl N-alkyipyrroles ayant 1 à 6 atomes de carbone; les vinyl-oxazoles; les vinyl-thiazoles; les vinylpyrrimidines; les vinylimidazoles; les vinyl cétones;
(vi) les amides (méth)acryliques obtenues à partir d'amines aliphatiques, linéaires, ramifiés, cycliques et/ou d'amines aromatiques, en C₁-C₂₀ telles que le tertiobutylacrylamide; les (méth)acrylamides tels que l'acrylamide, le méthacrylamide, les dialkyl(C₁-C₄)(méth)acrylamides ;
(vii) les oléfines tels que l'éthylène, le propylène, le styrène ou le styrène substitué;
(viii) les monomères acryliques ou vinyliques fluorés ou perfluorés, notamment les esters (méth)acryliques à motifs perfluoroalkyle;
(ix) les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyidiméthylammonium;
(x) les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium par exemple) ou par des sulfones cycliques (propane sulfone);
(xi) les (méth)acrylates ou (méth)acrylamides siliconés, notamment les esters(méth)acryliques à motifs siloxane;
(xii) leurs mélanges;
en présence
- d'un initiateur ayant au moins deux atomes et/ou groupes radicalairement transférables par polymérisation,
- d'un composé comprenant un métal de transition, susceptible de participer à une étape de réduction avec l'initiateur et une chaîne polymérique "dormante", choisi parmi ceux de formule Mⁿ⁺X'ₙ, dans laquelle :
- M est choisi parmi Cu, Au, Ag, Hg, Ni, Pd, Pt, Rh, Co, Ir, Fe, Ru, Os, Re, Mn, Cr, Mo, W, V, Nb, Ta et Zn, et
- X' représente un halogène (brome ou chlore notamment), OH, (O)_{1/2}, un radical alcoxy ayant 1-6 atomes de carbone, (SO₄)_{1/2}, (PO₄)_{1/3}, (HPO₄)_{1/2}, (H₂PO₄), un radical triflate, hexafluorophosphate, methanesulfonate, arylsulfonate, SeR, CN, NC, SCN, CNS, OCN, CNO, N₃ et R'CO₂, dans lequel R représente un radical aryle ou alkyle, linéaire ou ramifié, ayant 1-20, de préférence 1-10 atomes de carbone, et R' représente H ou un radical alkyle, linéaire ou ramifié, ayant 1-6 atomes de carbone, ou un radical aryle, éventuellement substitué par un ou plusieurs atomes d'halogènes notamment de fluor et/ou de chlore;
- n est la charge du métal;
et
- d'un ligand choisi parmi :
(i) les composés comprenant au moins un atome d'azote (N), d'oxygène (O), de phosphore (P) et/ou de soufre (S), susceptibles de se coordonner par une liaison σ audit composé comprenant un métal de transition;
(ii) les composés comprenant au moins deux atomes de carbone susceptibles de se coordonner par une liaison π audit composé comprenant un métal de transition;
(iii) les composés comprenant au moins un atome de carbone susceptibles de se coordonner par une liaison σ audit composé comprenant un métal de transition, mais qui ne forment pas de liaison carbone-carbone avec le monomère lors de la polymérisation;
(iv) les composés susceptibles de se coordonner par une liaison µ ou η audit composé comprenant un métal de transition;
ledit polymère comprenant un ou plusieurs monomères Mi dont l'homopolymère correspondant présente une Tg supérieure ou égale à 10°C, de préférence supérieure ou égale à 15°C, et encore mieux supérieure ou égale à 20°C; ce ou ces monomères Mi étant présents, dans le polymère final, en une quantité comprise entre 55 et 95% en poids, de préférence en une quantité de 60 à 93% en poids, et encore mieux en une quantité de 65-90% en poids, par rapport au poids total de monomères;
et ledit polymère comprenant par ailleurs, un ou plusieurs monomères Mj dont l'homopolymère correspondant présente une Tg inférieure ou égale à 10°C, de préférence inférieure ou égale à 5°C, et encore mieux inférieure ou égale à 0°C; ce ou ces monomères Mj étant présents, dans le polymère final, en une quantité comprise entre 5 et 45% en poids, de préférence en une quantité de 7 à 40% en poids, et encore mieux en une quantité de 10-35% en poids, par rapport au poids total de monomères.

2. Composition selon la revendication précédente, dans laquelle les monomères sont choisis parmi :
- les esters (méth)acryliques obtenus à partir d'alcools aliphatiques, linéaires ou ramifiés, de préférence en C₁-C₂₀;
- les esters (méth)acryliques en C₁-C₂₀ à motifs perfluoroalkyle;
- les esters(méth)acryliques en C₁-C₂₀ à motifs siloxane;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄)(méth)acrylamides ;
- les esters vinyliques, allyliques ou méthallyliques obtenus à partir d'alcools aliphatiques, linéaires ou ramifiés en C₁-C₁₀ ou cycliques en C₁-C₆ ;
- la vinylcaprolactame ;
- le styrène éventuellement substitué;
- leurs mélanges.

3. Composition selon l'une des revendications précédentes, dans laquelle l'initiateur est choisi parmi les composés de formule :
- R¹¹ₓR¹²_{y}R¹³_{z}C-(RX)ₜ dans laquelle x, y et z représentent un entier allant de 0 à 4, t un entier allant de 1 à 4, et x+y+z= 4-t;
- R¹³ₓC₆-(RX)_{y} (cycle à 6 carbones, saturé) dans laquelle x représente un entier allant de 7 à 11, y représente un entier allant de 1 à 5, et x+y= 12 ;
- R¹³ₓC₆-(RX)_{y} (cycle à 6 carbones, insaturé) dans laquelle x représente un entier allant de 0 à 5, y représente un entier allant de 1 à 6, et x+y= 6;
- -[-(R¹¹)(R¹²)(R¹³)C-(RX)-]ₙ dans laquelle n est supérieur ou égal à 1; cyclique ou linéaire;
- -[-(R12)ₓC₆(RX)_{y}-R¹¹-]ₙ dans laquelle x représente un entier allant de 0 à 6, y représente un entier allant de 1 à 6, et n est supérieur ou égal à 1, avec x+y=4 ou 6; cyclique ou linéaire;
- -[-(R¹²)ₓC₈(RX)_{y}-R¹¹-]ₙ dans laquelle x représente un entier allant de 0 à 12, y représente un entier allant de 1 à 12, et n est supérieur ou égal à 1, avec x+y=10 ou 12; cyclique ou linéaire;
- -[OSi(R¹¹)ₓ(RX)_{y}]ₙ, cyclique ou linéaire, dans laquelle x et y représentent un entier allant de 0 à 2, et n est supérieur ou égal à 1, avec x+y=2;
- R¹¹N-X₂
- (R¹¹)ₓP(O)_{y}-X₃₋ₓ dans laquelle x et y représentent des entiers allant de 0 à 2, et x+y = 5;
- (R¹¹O)ₓP(O)_{y}-X₃₋ₓ dans laquelle x et y représentent des entiers allant de 0 à 2, et x+y = 5;
- -[(R¹¹)ₜN_{z}P(O)ₓ(O-RX)_{y}-]ₙ, cyclique ou linéaire, dans laquelle x représente un entier allant de 0 à 4, y représente un entier allant de 1 à 5, z représente un entier allant de 0 à 2 et t représente un entier allant de 0 à 3, et n est supérieur ou égal à 1;
dans lesquelles :
- R, R¹¹, R¹² et R¹³ représentent, indépendamment l'un de l'autre, un atome d'hydrogène ou d'halogène; un radical alkyle, linéaire ou ramifié, ayant 1-20, de préférence 1-10 et plus préférentiellement 1-6 atomes de carbone; un radical cycloalkyle ayant 3-8 atomes de carbone; un radical -C(=Y)R⁵, -C(=Y)NR⁶R⁷ ou -R⁸₃Si; -COCl, -OH, -CN, un radical alkényle ou alkynyle ayant 2-20, de préférence 2-6, atomes de carbone; un radical oxiranyle, glycidyle, alkylène ou alkénylène substitué avec un oxiranyle ou un glycidyle; un radical aryle, heterocyclyle, aralkyle, aralkenyle; un radical alkyle ayant 1-6 atomes de carbone dans lequel tout ou partie des atomes d'hydrogène sont substitués soit par des atomes d'halogènes tels que fluor, chlore ou brome, soit par un groupe alcoxy ayant 1-4 atomes de carbone ou par un radical aryle, heterocyclyle, -C(=Y)R⁵, -C(=Y)NR⁶R⁷, oxiranyle, glycidyle;
- X représente un atome d'halogène tel que Cl, Br, I, ou un radical -OR', -SR, -SeR, -OC(=O)R', -OP(=O)R- -OP(=O)(OR')₂, -OP(=O)OR', -O-NR'₂, -S-C(=S)NR'₂, -CN, -NC, -SCN, -CNS, -OCN, -CNO et -N₃, dans lequel R' représente un radical alkyle ayant 1-20 atomes de carbone éventuellement substitué par un ou plusieurs atomes d'halogènes notamment de fluor et/ou de chlore; et R représente un radical aryle ou alkyle, linéaire ou ramifié, ayant 1-20, de préférence 1-10, atomes de carbone; le groupement -NR'₂ pouvant en outre représenter un groupement cyclique, les deux groupes R' étant joints de manière à former un hétérocycle à 5, 6 ou 7 membres;
- Y représente O, S ou NR⁸ (de préférence O),
- R⁵ représente un radical alkyle, alkylthio, alcoxy, linéaire ou ramifié, ayant 1-20 atomes de carbone; un radical OH; un radical OM' avec M' = métal alcalin; un radical aryloxy ou un radical heterocyclyloxy;
- R⁶ et R⁷ représentent, indépendamment l'un de l'autre, H ou un radical alkyle, linéaire ou ramifié, ayant 1-20 atomes de carbone; étant donné que R⁶ et R⁷ peuvent être joints pour former un groupe alkylène ayant 2-7, de préférence 2-5, atomes de carbone;
- R⁸ représente H; un radical alkyle, linéaire ou ramifié, ayant 1-20 atomes de carbone ou un radical aryle.

4. Composition selon l'une des revendications précédentes, dans laquelle l'initiateur est choisi parmi :
- l'octa-2-isobutyrylbromide-octatertiobutyl-calix(8)arène,
- l'octa-2-propionylbromide-octatertiobutyl-calix(8)arène, et
- l'hexakis α-bromométhylbenzène.

5. Composition selon l'une des revendications précédentes, dans laquelle le ligand est choisi parmi :
(i) les composés de formule : R⁹-Z-(R¹⁴-Z)ₘ-R¹⁰ ou R²⁰R²¹C[C(=Y)R⁵]
dans lesquelles :
- R⁹ et R¹⁰ sont, indépendamment l'un de l'autre, un atome d'hydrogène; un radical alkyle, linéaire ou ramifié, ayant 1-20, de préférence 1-10 atomes de carbone; un radical aryle; un radical hétérocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et(ou YC(=Y)R⁸, R⁵ à R⁸ et Y ayant les définitions données en revendication 3; étant donné que R⁹ et R¹⁰ peuvent être joints de manière à former un cycle, saturé ou insaturé;
- R¹⁴ représente, indépendamment les uns des autres, un groupe divalent choisi parmi les alcanediyles ayant 2-4 atomes de carbone; les alkénylènes ayant 2-4 atomes de carbone; les cycloalcanediyles ayant 3-8 atomes de carbone; les cycloalkènediyles ayant 3-8 atomes de carbone; les arènediyles et les hétérocyclylènes;
- Z représente O, S, NR¹⁵ ou PR¹⁵ avec R¹⁵ représentant H; un radical alkyle, linéaire ou ramifié, ayant 1-20 atomes de carbone; un radical aryle; un radical heterocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸, R⁵ à R⁸ et Y ayant les définitions données dans la revendication 3;
- m est compris entre 0 et 6;
- R²⁰ et R²¹ sont, indépendamment l'un de l'autre, un atome d'hydrogène; un atome d'halogène; un radical alkyle, linéaire ou ramifié, ayant 1-20, de préférence 1-10 atomes de carbone; un radical aryle; un radical hétérocyclyle; étant donné que R²⁰ et R²¹ peuvent être joints de manière à former un cycle, saturé ou insaturé; étant donné que chaque radical peut en outre être substitué avec un radical alkyle ayant 1-6 atomes de carbone, un radical alcoxy ayant 1-6 atomes de carbone ou un radical aryle;
(ii) le monoxyde de carbone; les porphyrines et les porphycènes, éventuellement substitués; l'éthylènediamine et le propylènediamine, éventuellement substitués; les multiamines avec amines tertiaires telles que le pentaméthyldiéthylènetriamine; les aminoalcools tels que l'aminoéthanol et l'aminoprpoanol, éventuellement substitués; les glycols tels que l'éthylèneglycol ou le propylèneglycol, éventuellement substitués; les arènes tels que le benzène, éventuellement substitués; le cyclopentadiène, éventuellement substitué; les pyridines et bipyridines, éventuellement substituées; l'acétonitrile; la 1,10-phénanthroline; les cryptands et les éthers-couronnes; la spartéine.

6. Composition selon l'une des revendications précédentes, dans laquelle l'agent propulseur est choisi parmi, seul ou en mélange, les hydrocarbures volatils tels que le n-butane, le propane, l'isobutane, le pentane, un hydrocarbure chloré et/ou fluoré; le gaz carbonique, le protoxyde d'azote, le diméthyléther (DME), l'azote, l'air comprimé.

7. Composition selon l'une des revendications précédentes, dans laquelle l'agent auxiliaire de filmification est choisi parmi, seul ou en mélange :
- les glycols et leurs dérivés tels que le diéthylène glycol éthyléther, le diéthylène glycol méthyléther, le diéthylène glycol butyléther, le diéthylène glycol hexyléther, l'éthylène glycol éthyléther, l'éthylène glycol butyléther, l'éthylène glycol hexyléther;
- les esters de glycérol, tels que le diacétate de glycérol et le triacétate de glycérol;
- les dérivés de propylène glycol et en particulier le propylène glycol phényléther, le propylène glycol diacétate, le propylène glycol méthyléther, le propylène glycol éthyléther, le propylène glycol butyléther, le dipropylène glycol méthyléther, le dipropylène glycol butyléther, le dipropylène glycol éthyléther, le tripropylène glycol butyléther, le tripropylène glycol méthyléther;
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone oxyéthylénées.

8. Composition selon l'une des revendications précédentes, dans laquelle le polymère de structure 'en étoiles' est présent en une quantité comprise entre 1-95% en poids de matière sèche, par rapport au poids total de la composition, de préférence entre 1-50% en poids et préférentiellement entre 1-20% en poids.

9. Composition selon l'une des revendications précédentes, dans laquelle le polymère de structure 'en étoiles' est présent dans le milieu sous forme dissoute ou en dispersion, dans une phase aqueuse, organique ou hydroorganique notamment alcoolique ou hydroalcoolique.

10. Composition selon l'une des revendications précédentes, se présentant sous la forme d'un spray, d'une laque, d'une mousse, de crème, de gel, d'émulsion, de lotion ou de cire.

11. Procédé de maintien ou de mise en forme des cheveux, **caractérisé par le fait qu'**il consiste à appliquer sur ceux-ci une composition cosmétique selon l'une des revendications précédentes.

12. Utilisation d'une composition cosmétique selon l'une des revendications 1 à 10, pour la fabrication d'un produit cosmétique capillaire destiné à maintenir et/ou fixer et/ou traiter la coiffure et/ou les cheveux.

13. Utilisation selon la revendication 12, pour la fabrication d'un produit de coiffage tel qu'une laque, un spray ou une mousse, en vue d'obtenir le maintien ou la mise en forme de la coiffure.

## Claims

1. Hair cosmetic composition, in particular for the treatment and/or the fixing of the hair, comprising, in a cosmetically acceptable medium comprising at least one constituent chosen from water; one or more cosmetically acceptable solvents, such as alcohols comprising 1 to 4 carbon atoms, ethers, ketones or C₁-C₃ lower carboxylic acid esters; cosmetic active principles; softeners; antioxidants; opacifiers; antifoaming agents; preservatives; sequestering agents; UV screening agents; ceramides; antidandruff agents; complexing agents; agents for dramatic hair loss; antifungal or antiseptic agents; thickeners; fixing or nonfixing polymers; conditioners; propellants; basifying or acidifying agents; hydrophilic polymers; film-forming polymers, in particular in aqueous dispersion; surfactants, in particular anionic or nonionic surfactants, optionally comprising silicone groups; or an additional agent which is able to form a film, such as a plasticizing agent and/or a coalescence agent;
at least one polymer with a "star" structure capable of being obtained by atom transfer radical polymerization.
- of one or more radically polymerizable monomers, chosen from:
(i) acrylic or methacrylic esters obtained from linear, branched or cyclic aliphatic alcohols and/or from aromatic alcohols comprising 1 to 20 carbon atoms, such as methyl (meth)acrylate, ethyl (meth)acrylate, propyl (meth)acrylate, butyl (meth)acrylate, isobutyl (meth)acrylate or tert-butyl (meth)acrylate;
(ii) C₁-C₄ hydroxyalkyl (meth)acrylates, such as 2-hydroxyethyl (meth)acrylate, or 2-hydroxypropyl (meth)acrylate;
(iii) ethylene glycol, diethylene glycol or polyethylene glycol (meth)acrylates with a hydroxyl or ether end;
(iv) vinyl, allyl or methallyl esters obtained from linear or branched C₁-C₁₀ or cyclic C₁-C₆ aliphatic alcohols and/or from C₁-C₆ aromatic alcohols, such as vinyl acetate, vinyl propionate, vinyl benzoate or vinyl tert-butylbenzoate;
(v) N-vinylpyrrolidone; vinylcaprolactam; vinyl-N-alkylpyrroles having 1 to 6 carbon atoms; vinyloxazoles; vinylthiazoles; vinylpyrimidines; vinylimidazoles; or vinyl ketones;
(vi) (meth)acrylamides obtained from linear, branched or cyclic aliphatic amines and/or from aromatic amines comprising 1 to 20 carbon atoms, such as tert-butylacrylamide; or (meth)acrylamides, such as acrylamide, methacrylamide or di(C₁-C₄)alkyl(meth)acrylamides;
(vii) olefins, such as ethylene, propylene, styrene or substituted styrene;
(viii) fluorinated or perfluorinated acrylic or vinyl monomers, in particular (meth)acrylic esters with perfluoroalkyl units;
(ix) monomers comprising an amine functional group in the free or else partially or completely neutralized or else partially or completely quaternized form, such as dimethylaminoethyl (meth)acrylate, dimethylaminoethylmethacrylamide, vinyl amine, vinylpyridine or diallyldimethylammonium chloride;
(x) carboxybetaines or sulphobetaines obtained by partial or complete quaternization of monomers comprising ethylenic unsaturation comprising an amine functional group by sodium salts of carboxylic acids comprising a mobile halide (sodium chloroacetate, for example) or by cyclic sulphones (propane sulphone);
(xi) silicone-comprising (meth)acrylates or (meth)acrylamides, in particular (meth)acrylic esters comprising siloxane units;
(xii) their mixtures;
in the presence
- of an initiator having at least two atoms and/or groups radically transferable by polymerization,
- of a compound comprising a transition metal, capable of participating in a reduction stage with the initiator and a "dormant" polymer chain, chosen from those of formula Mⁿ⁺X'ₙ, in which:
- M is chosen from Cu, Au, Ag, Hg, Ni, Pd, Pt, Rh, Co, Ir, Fe, Ru, Os, Re, Mn, Cr, Mo, W, V, Nb, Ta and Zn, and
- X' represents a halogen (in particular bromine or chlorine), OH, (O)_{1/2}, an alkoxy radical having 1-6 carbon atoms, (SO₄)_{1/2}, (PO₄)_{1/3}, (HPO₄)_{1/2}, (H₂PO₄) and a triflate, hexafluorophosphate, methanesulphonate, arylsulphonate, SeR, CN, NC, SCN, CNS, OCN, CNO, N₃ and R'CO₂, in which R represents an aryl or linear or branched alkyl radical having 1-20, preferably 1-10, carbon atoms and R' represents H or a linear or branched alkyl radical having 1-6 carbon atoms or an aryl radical which is optionally substituted by one or more halogen atoms, in particular fluorine and/or chlorine atoms;
- n is the charge on the metal;
and
- of a ligand chosen from:
(i) compounds comprising at least one nitrogen (N), oxygen (O) phosphorus (P) and/or sulphur (S) atom which are capable of coordinating via a σ bond to the said compound comprising a transition metal;
(ii) compounds comprising at least two carbon atoms capable of coordinating via a π bond to the said compound comprising a transition metal;
(iii) compounds comprising at least one carbon atom capable of coordinating via a σ bond to the said compound comprising a transition metal but which do not form a carbon-carbon bond with the monomer during the polymerization;
(iv) compounds capable of coordinating via a µ or η bond to the said compound comprising a transition metal;
the said polymer comprising one or more monomers Mi, the corresponding homopolymer of which exhibits a Tg of greater than or equal to 10°C, preferably greater than or equal to 15°C and even better still of greater than or equal to 20°C; this or these monomers Mi being present, in the final polymer, in an amount of between 55 and 95% by weight, preferably in an amount of 60 to 93% by weight and even better still in an amount of 65 to 90% by weight, with respect to the total weight of monomers;
and the said polymer furthermore comprising one or more monomers Mj, the corresponding homopolymer of which exhibits a Tg of less than or equal to 10°C, preferably of less than or equal to 5°C and even better still of less than or equal to 0°C; this or these monomers Mj being present, in the final polymer, in an amount of between 5 and 45% by weight, preferably in an amount of 7 to 40% by weight and even better still in an amount of 10-35% by weight, with respect to the total weight of monomers.

2. Composition according to the preceding claim, in which the monomers are chosen from:
- (meth)acrylic esters obtained from linear or branched aliphatic alcohols, preferably C₁-C₂₀ alcohols;
- C₁-C₂₀ (meth)acrylic esters comprising perfluoroalkyl units;
- C₁-C₂₀ (meth)acrylic esters comprising siloxane units;
- (meth)acrylamides obtained from linear, branched or cyclic aliphatic amines and/or from aromatic amines preferably comprising 1 to 20 carbon atoms, such as tert-butylacrylamide; or (meth)acrylamides, such as acrylamide, methacrylamide or di(C₁-C₄)alkyl(meth)acrylamides;
- vinyl, allyl or methallyl esters obtained from linear or branched C₁-C₁₀ or cyclic C₁-C₆ aliphatic alcohols;
- vinylcaprolactam;
- optionally substituted styrene;
- their mixtures.

3. Composition according to one of the preceding claims, in which the initiator is chosen from the compounds of formula:
- R¹¹ₓR¹²_{y}R¹³_{z}C-(RX)ₜ, in which x, y and z represent an integer ranging from 0 to 4, t an integer ranging from 1 to 4, and x+y+z = 4-t;
- R¹³ₓC₆-(RX)_{y} (saturated ring with 6 carbons), in which x represents an integer ranging from 7 to 11, y represents an integer ranging from 1 to 5, and x+y = 12;
- R¹³ₓC₆-(RX)_{y} (unsaturated ring with 6 carbons), in which x represents an integer ranging from 0 to 5, y represents an integer ranging from 1 to 6, and x+y = 6;
- -[-(R¹¹)(R¹²)(R¹³)C-(RX)-]ₙ, in which n is greater than or equal to 1; cyclic or linear;
- -[-(R¹²)ₓC₆(Rx)_{y}-R¹¹-]ₙ, in which x represents an integer ranging from 0 to 6, y represents an integer ranging from 1 to 6 and n is greater than or equal to 1, with x+y = 4 or 6; cyclic or linear;
- -[-(R¹²)ₓC₆(RX)_{y}-R¹¹-]ₙ, in which x represents an integer ranging from 0 to 12, y represents an integer ranging from 1 to 12 and n is greater than or equal to 1, with x+y = 10 or 12; cyclic or linear;
- -[OSi(R¹¹)ₓ(RX)_{y}]ₙ, cyclic or linear, in which x and y represent an integer ranging from 0 to 2 and n is greater than or equal to 1, with x+y = 2;
- R¹¹N-X₂
- (R¹¹)ₓP(O)_{y}-X₃₋ₓ, in which x and y represent integers ranging from 0 to 2 and x+y = 5;
- (R¹¹O)ₓP(O)_{y}-X₃₋ₓ, in which x and y represent integers ranging from 0 to 2 and x+y = 5;
- -[(R¹¹)ₜN_{z}P(O)ₓ(O-RX)_{y}-]ₙ, cyclic or linear, in which x represents an integer ranging from 0 to 4, y represents an integer ranging from 1 to 5, z represents an integer ranging from 0 to 2, t represents an integer ranging from 0 to 3 and n is greater than or equal to 1;
in which:
- R_{,} R¹¹, R¹² and R¹³ represent, independently of one another, a hydrogen or halogen atom; a linear or branched alkyl radical having 1-20, preferably 1-10 and more preferably 1-6 carbon atoms; a cycloalkyl radical having 3-8 carbon atoms; a -C(=Y)R⁵, -C(=Y)NR⁶R⁷ or -R⁸₃Si radical; -COCl; -OH; -CN; an alkenyl or alkynyl radical having 2-20, preferably 2-6, carbon atoms; an oxiranyl or glycidyl radical or an alkylene or alkenylene radical substituted with an oxiranyl or a glycidyl; an aryl, heterocyclyl, aralkyl or aralkenyl radical; or an alkyl radical having 1-6 carbon atoms in which all or part of the hydrogen atoms are substituted either by halogen atoms, such as fluorine, chlorine or bromine, or by an alkoxy group having 1-4 carbon atoms or by an aryl, heterocyclyl, -C(=Y)R⁵, -C(=Y)NR⁶R⁷, oxiranyl or glycidyl radical;
- X represents a halogen atom, such as Cl, Br or I, or an -OR', -SR, -SeR, -OC(=O)R', -OP(=O)R', -OP(=O)(OR')₂, -OP(=O)OR', -O-NR'₂, -S-C(=S)NR'₂, -CN, -NC, -SCN, -CNS, -OCN, -CNO or -N₃ radical, in which R' represents an alkyl radical having 1-20 carbon atoms which is optionally substituted by one or more halogen atoms, in particular fluorine and/or chlorine atoms, and R represents a linear or branched alkyl or aryl radical having 1-20, preferably 1-10, carbon atoms, it additionally being possible for the -NR'₂ group to represent a cyclic group, the two R' groups being joined so as to form a 5-, 6- or 7-membered heterocycle;
- Y represents O, S or NR⁸ (preferably O);
- R⁵ represents a linear or branched alkyl, alkylthio or alkoxy radical having 1-20 carbon atoms; an OH radical; an OM' radical with M' = alkali metal; an aryloxy radical or a heterocyclyloxy radical;
- R⁶ and R⁷ represent, independently of one another, H or a linear or branched alkyl radical having 1-20 carbon atoms; it being given that R⁶ and R⁷ can be joined to form an alkylene group having 2-7, preferably 2-5, carbon atoms;
- R⁸ represents H; a linear or branched alkyl radical having 1-20 carbon atoms or an aryl radical.

4. Composition according to one of the preceding claims, in which the initiator is chosen from:
- octa(2-isobutyrylbromide)-octa (tert-butyl)calix(8)arene,
- octa(2-propionylbromide)-octa(tert-butyl)calix(8)arene, and
- hexakis(α-bromomethyl)benzene.

5. Composition according to one of the preceding claims, in which the ligand is chosen from:
(i) the compounds of formula: R⁹-Z-(R¹⁴-Z)ₘ-R¹⁰ or R²⁰R²¹C[C(=Y)R⁵]
in which:
- R⁹ and R¹⁰ are, independently of one another, a hydrogen atom; a linear or branched alkyl radical having 1-20, preferably 1-10, carbon atoms; an aryl radical; a heterocyclyl radical; or an alkyl radical having 1-6 carbon atoms which is substituted with an alkoxy radical having 1-6 carbon atoms or a dialkylamino radical having 1-4 carbon atoms or a -C(=Y)R⁵ or -C(=Y)NR⁶R⁷ and/or YC(=Y)R⁸ radical, R⁵ to R⁸ and Y having the definitions given in Claim 3; it being given that R⁹ and R¹⁰ can be joined so as to form a saturated or unsaturated ring;
- R¹⁴ represents, independently of one another, a divalent group chosen from alkanediyls having 2-4 carbon atoms; alkenediyls having 2-4 carbon atoms; cycloalkanediyls having 3-8 carbon atoms; cycloalkenediyls having 3-8 carbon atoms; arenediyls and heterocyclylenes;
- Z represents O, S, NR¹⁵ or PR¹⁵, with R¹⁵ representing H; a linear or branched alkyl radical having 1-20 carbon atoms; an aryl radical; a heterocyclyl radical; or an alkyl radical having 1-6 carbon atoms which is substituted with an alkoxy radical having 1-6 carbon atoms or a dialkylamino radical having 1-4 carbon atoms or a -C(=Y)R⁵ or -C(=Y)NR⁶R⁷ and/or YC(=Y)R⁸ radical (R⁵ to R⁸ and Y having the definitions given in Claim 3;
- m is between 0 and 6;
- R²⁰ and R²¹ are, independently of one another, a hydrogen atom; a halogen atom; a linear or branched alkyl radical having 1-20, preferably 1-10, carbon atoms; an aryl radical; or a heterocyclyl radical; it being given that R²⁰ and R²¹ can be joined so as to form a saturated or unsaturated ring; it being given that, in addition, each radical can be substituted with an alkyl radical having 1-6 carbon atoms, an alkoxy radical having 1-6 carbon atoms or an aryl radical;
(ii) carbon monoxide; optionally substituted porphyrins and porphycenes; optionally substituted ethylenediamine and propylenediamine; polyamines with tertiary amines, such as pentamethyldiethylenetriamine; aminoalcohols, such as aminoethanol and aminopropanol, which are optionally substituted; glycols, such as ethylene glycol or propylene glycol, which are optionally substituted; arenes, such as benzene, which are optionally substituted; optionally substituted cyclopentadiene; optionally substituted pyridines and bipyridines; acetonitrile; 1,10-phenanthroline; cryptands and crown ethers; or sparteine.

6. Composition according to one of the preceding claims, in which the propellant is chosen from, alone or as a mixture, volatile hydrocarbons, such as n-butane, propane, isobutane, pentane or a chlorinated and/or fluorinated hydrocarbon; carbon dioxide gas, nitrous oxide, dimethyl ether (DME), nitrogen or compressed air.

7. Composition according to one of the preceding claims, in which the additional agent which is able to form a film is chosen from, alone or as a mixture:
- glycols and their derivatives, such as diethylene glycol ethyl ether, diethylene glycol methyl ether, diethylene glycol butyl ether, diethylene glycol hexyl ether, ethylene glycol ethyl ether, ethylene glycol butyl ether or ethylene glycol hexyl ether;
- glycerol esters, such as glyceryl diacetate and glyceryl triacetate;
- propylene glycol derivatives and in particular propylene glycol phenyl ether, propylene glycol diacetate, propylene glycol methyl ether, propylene glycol ethyl ether, propylene glycol butyl ether, dipropylene glycol methyl ether, dipropylene glycol butyl ether, dipropylene glycol ethyl ether, tripropylene glycol butyl ether or tripropylene glycol methyl ether;
- acid esters, in particular carboxylic acid esters, such as citrates, phthalates, adipates, carbonates, tartrates, phosphates or sebacates,
- oxyethylenated derivatives, such as oxyethylenated oils, in particular vegetable oils, such as caster oil; or oxyethylenated silicone oils.

8. Composition according to one of the preceding claims, in which the polymer with the "star" structure is present in an amount of between 1-95% by weight on a dry basis, with respect to the total weight of the composition, preferably between 1-50% by weight and preferentially between 1-20% by weight.

9. Composition according to one of the preceding claims, in which the polymer with the "star" structure is present in the medium in the form dissolved or in dispersion in an aqueous, organic or aqueous/organic phase, in particular an alcoholic or aqueous/alcoholic phase.

10. Composition according to one of the preceding claims, which is provided in the form of a spray, lacquer, foam, cream, gel, emulsion, lotion or wax.

11. Process for the form retention or shaping of the hair, **characterized in that** it consists in applying, to the latter, a cosmetic composition according to one of the preceding claims.

12. Use of a cosmetic composition according to one of Claims 1 to 10 in the manufacture of a hair cosmetic product intended to retain the form of and/or fix and/or treat the hairstyle and/or the hair.

13. Use according to Claim 12 in the manufacture of a styling product, such as a lacquer, a spray or a foam, for the purpose of obtaining form retention or shaping of the hairstyle.

## Patentansprüche

1. Kosmetische Zusammensetzung für das Haar, insbesondere zur Behandlung und/oder Fixierung der Haare, die in einem kosmetisch akzeptablen Medium mindestens einen Bestandteil, der unter den folgenden Verbindungen ausgewählt ist: Wasser; einem oder mehreren kosmetisch akzeptablen Lösungsmitteln, wie C₁₋₄-Alkoholen, Ethern, Ketonen, Estern von niederen Carbonsäuren mit 1 bis 3 Kohlenstoffatomen; kosmetischen Wirkstoffen; reizlindernden Wirkstoffen, Antioxidantien, Trübungsmitteln, Schaumverhütungsmitteln, Konservierungsmitteln, Maskierungsmitteln, UV-Filtern, Ceramiden; Antischuppenmitteln; Komplexbildnern; Wirkstoffen gegen Haarausfall; antimykotischen oder antiseptischen Wirkstoffen; Verdickungsmitteln, fixierenden oder nichtfixierenden Polymeren oder Konditioniermitteln; Treibmitteln, Alkalisierungsmitteln oder Ansäuerungsmitteln; hydrophilen Polymeren; filmbildenden Polymeren, insbesondere in wässriger Dispersion; grenzflächenaktiven Stoffen und insbesondere anionischen oder nichtionischen grenzflächenaktiven Stoffen, die ggf. siliconhaltig sind; Hilfsmitteln zur Filmbildung wie Weichmachern und/oder Koaleszenzmitteln;
und mindestens ein Polymer mit "sternförmiger" Struktur enthält, das erhältlich ist durch radikalische Atomtransferpolymerisation:
- eines oder mehrerer radikalisch polymerisierbarer Monomere, die ausgewählt sind unter:
(i) den Acrylestern oder Methacrylestern, die ausgehend von aliphatischen, geradkettigen, verzweigten, cyclischen und/oder aromatischen Alkoholen vorzugsweise mit 1 bis 20 Kohlenstoffatomen hergestellt werden, wie beispielsweise Methyl(meth)acrylat, Ethyl(meth)-acrylat, Propyl(meth)acrylat, Butyl(meth)acrylat, Isobutyl(meth)acrylat und t-Butyl(meth)acrylat;
(ii) C₁₋₄-Hydroxyalkyl(meth)acrylaten, wie 2-Hydroxyethyl(meth)acrylat oder 2-Hydroxypropyl(meth)acrylat;
(iii) Ethylenglykol(meth)acrylaten, Diethylenglykol(meth)acrylaten und Polyethylenglykol(meth)-acrylaten mit endständigen Hydroxy- oder Ethergruppen;
(iv) Vinylestern, Allylestern oder Methallylestern, die ausgehend von aliphatischen, geradkettigen oder verzweigten C₁₋₁₀-Alkoholen oder cyclischen C₁₋₆-Alkohlen und/oder aromatischen Alkoholen mit 1 bis 6 Kohlenstoffatomen hergestellt werden, beispielsweise Vinylacetat, Vinylpropionat, Vinylbenzoat und Vinyl-*t*-butylbenzoat;
(v) N-Vinylpyrrolidon; Vinylcaprolactam; Vinyl-N-alkylpyrrolen mit 1 bis 6 Kohlenstoffatomen; Vinyloxazolen, Vinylthiazolen; Vinylpyrimidinen; Vinylimidazolen; und Vinylketonen;
(vi) (Meth)acrylamiden, die ausgehend von aliphatischen, geradkettigen, verzweigten, cyclischen und/oder aromatischen Aminen vorzugsweise mit 1 bis 20 Kohlenstoffatomen hergestellt werden, wie *t*-Butylacrylamid; (Meth)acrylamiden, wie Acrylamid, Methacrylamid und Di-C₁₋₄-alkyl(meth)acrylamiden;
(vii) Olefinen, wie Ethylen, Propylen, Styrol oder substituiertem Styrol;
(viii) fluorierten oder perfluorierten Acrylmonomeren oder Vinylmonomeren, insbesondere (Meth)acrylestern mit Perfluoralkylgruppen;
(ix) Monomeren, die eine Aminogruppe in freier Form oder ganz oder teilweise neutralisiert oder ganz oder teilweise quaternisiert enthalten, wie Dimethylaminoethyl(meth)-acrylat, Dimethylaminoethylmethacrylamid, Vinylamin, Vinylpyridin und Diallyldimethylammoniumchlorid;
(x) Carboxybetainen oder Sulfobetainen, die durch partielle oder vollständige Quaternisierung von Monomeren mit ethylenischer Doppelbindung, die eine Aminogruppe enthalten, durch Natriumsalze von Carbonsäuren mit mobilem Halogenid (beispielsweise Natriumchloracetat) oder durch cyclische Sulfone (Propansulfon) hergestellt werden;
(xi) siliconhaltigen (Meth)acrylaten oder (Meth)acrylamiden, insbesondere den (Meth)acrylestem mit Siloxangruppen; und
(xii) deren Gemischen.
in Gegenwart
- eines Initiators, der mindestens zwei durch Polymerisation radikalisch transferierbare Atome und/oder Gruppen aufweist,
- einer Verbindung, die ein Übergangsmetall enthält, das an einem Reduktionsschritt mit dem Initiator und einer "schlafenden" Polymerkette teilnehmen kann und die unter den Verbindungen der Formel Mⁿ⁺X'ₙ ausgewählt ist, worin:
- M unter Cu, Au, Ag, Hg, Ni, Pd, Pt, Rh, Co, Ir, Fe, Ru, Os, Re, Mn, Cr, Mo, W, V, Nb, Ta und Zn ausgewählt ist,
- X' ein Halogen (insbesondere Brom oder Chlor), OH, (O)_{1/2}, eine Alkoxygruppe mit 1 bis 6 Kohlenstoffatomen, (SO4)_{1/2}, (PO4)_{1/3}, (HPO4)_{1/2}, (H2PO4), eine Triflatgruppe, Hexafluorphosphat, Methansulfonat, Arylsulfonat, SeR, CN, NC, SCN, CNS, OCN, CNO, N3 et R'CO₂ bedeuten kann, wobei R eine geradkettige oder verzweigte Aryl- oder Alkylgruppe mit 1 bis 20 und vorzugsweise 1 bis 10 Kohlenstoffatomen ist und R' H oder eine geradkettige oder verzweigte Alkylgruppe mit 1 bis 6 Kohlenstoffatomen oder eine Arylgruppe bedeutet, die ggf. mit einem oder mehreren Halogenatomen und insbesondere Fluor und/oder Chlor substituiert ist; und
- n die Ladung des Metalls bedeutet; und
- eines Liganden, der ausgewählt ist unter:
(i) den Verbindungen, die mindestens ein Stickstoffatom (N), Sauerstoffatom (O), Phosphoratom (P) und/oder Schwefelatom (S) enthalten und die über eine σ-Bindung mit der Verbindung, die ein Übergangsmetall enthält, eine Koordinationsverbindung bilden können;
(ii) Verbindungen, die mindestens zwei Kohlenstoffatome aufweisen und die über eine π-Bindung mit der Verbindung, die ein Übergangsmetall enthält, eine Koordinationsverbindung bilden können;
(iii) Verbindungen, die mindestens ein Kohlenstoffatom enthalten und die über eine σ-Bindung mit der Verbindung, die ein Übergangsmetall enthält, eine Koordinationsverbindung bilden können, die jedoch mit dem Monomer bei der Polymerisation keine Kohlenstoff-Kohlenstoff-Bindung ausbilden; und
(iv) Verbindungen, die über eine µ- oder η-Bindung mit der Verbindung, die ein Übergangsmetall enthält, eine Koordinationsverbindung bilden können;
wobei das Polymer ein oder mehrere Monomere Mi enthält, deren korrespondierendes Homopolymer eine Tg von 10°C oder darüber, vorzugsweise mindestens 15°C und noch besser mindestens 20°C aufweist; wobei das Monomer Mi oder die Monomere Mi in dem fertigen Polymer in einer Menge von 55-95 Gew.-%, vorzugsweise 60-93 Gew.-% und noch besser 65-90 Gew.-%, bezogen auf das Gesamtgewicht der Monomere, vorliegen; und
wobei das Polymer im Übrigen ein oder mehrere Monomere Mj enthält, deren korrespondierendes Homopolymer eine Tg von 10°C oder darunter, vorzugsweise höchstens 5°C und noch besser höchstens 0°C aufweist; wobei das Monomer Mj oder die Monomere Mj in dem fertigen Polymer in einer Menge von 5-45 Gew.-%, vorzugsweise in einer Menge von 7-40 Gew.-% und noch besser in einer Menge von 10-35 Gew.-%, bezogen auf das Gesamtgewicht der Monomere, vorliegt.

2. Zusammensetzung nach dem vorhergehenden Anspruch, wobei die Monomere ausgewählt sind unter:
- (Meth)acrylestern, die ausgehend von aliphatischen, geradkettigen oder verzweigten Alkoholen mit vorzugsweise mit 1 bis 20 Kohlenstoffatomen hergestellt werden;
- (Meth)acrylestern mit 1 bis 20 Kohlenstoffatomen, die Perfluoralkylgruppen enthalten;
- (Meth)acrylestern mit 1 bis 20 Kohlenstoffatomen, die Siloxangrüppen enthalten;
- (Meth)acrylamiden, die ausgehend von aliphatischen, geradkettigen, verzweigten, cyclischen und/oder aromatischen Aminen, vorzugsweise mit 1 bis 20 Kohlenstoffatomen, hergestellt werden, wie t-Butylacrylamid; (Meth)acrylamiden, wie Acrylamid, Methacrylamid, Di-C₁₋₄-alkyl(meth)acrylamiden;
- Vinylestern, Allylestern oder Methallylestern, die ausgehend von aliphatischen, geradkettigen oder verzweigten C₁₋₁₀-Alkoholen oder cyclischen C₁₋₆-Alkohlen hergestellt werden;
- Vinylcaprolactam;
- ggf. substituiertem Styrol; und
- deren Gemischen.

3. Zusammensetzung nach einem der vorhergehenden Ansprüche, **dadurch gekennzeichnet, dass** der Initiator unter den Verbindungen der folgenden Formeln ausgewählt ist:
- R¹¹ₓR¹²_{y}R¹³_{z}C-(RX)ₜ, in der x, y und z 0 oder eine ganze Zahl von 1 bis 4 bedeuten, t eine ganze Zahl von 1 bis 4 ist und x+y+z=4-t;
- R¹³ₓC₆-(RX)_{y} (gesättigter Ring mit 6 Kohlenstoffatomen), wobei x eine ganze Zahl von 7 bis 11 bedeutet, y eine ganze Zahl von 1 bis 5 ist und x+y=12;
- R¹³ₓC₆-(RX)_{y} (ungesättigter Ring mit 6 Kohlenstoffatomen), wobei x 0 oder eine ganze Zahl von 1 bis 5 bedeutet, y eine ganze Zahl von 1 bis 6 ist und x+y=6;
- [-R¹¹)(R¹²)(R¹³)C-(RX)-]ₙ, wobei n 1 bedeutet oder darüber liegt und die Verbindung cyclisch oder geradkettig vorliegt;
- [-(R¹²)ₓC₆(RX)_{y}-R¹¹-]ₙ, wobei x 0 oder eine ganze Zahl von 1 bis 6 bedeutet, y eine ganze Zahl von 1 bis 6 ist, und n 1 bedeutet oder darüber liegt, mit x+y=4 oder 6; wobei die Verbindung cyclisch oder geradkettig ist;
- [-(R¹²)ₓC₆(RX)_{y}-R¹¹-]ₙ, wobei x 0 oder eine ganze Zahl von 1 bis 12 bedeutet, y eine ganze Zahl von 1 bis 12 ist, und n 1 bedeutet oder darüber liegt, mit x+y=10 oder 12; wobei die Verbindung cyclisch oder geradkettig ist;
- -[OSi(R¹¹)ₓ(RX)_{y}]ₙ, wobei die Verbindung cyclisch oder geradkettig vorliegt und wobei x und y 0 oder eine ganze Zahl von 1 bis 2 bedeuten und n 1 ist oder darüber liegt, mit x+y=2;
- R¹¹N-X₂
- (R¹¹)ₓP(O)_{y}-X₃₋ₓ, wobei x und y 0 oder ganze Zahlen von 1 bis 2 bedeuten und x+y=5;
- (R11O)ₓP(O)_{y}-X₃₋ₓ, wobei x und y 0 oder ganze Zahlen von 1 bis 2 bedeuten, und x+y=5;
- -[(R¹¹)ₜN_{z}P(O)ₓ(O-RX)_{y}-]ₙ, wobei die Verbindung cyclisch oder geradkettig vorliegt und wobei x 0 oder eine ganze Zahl von 1 bis 4 bedeutet, y eine ganze Zahl von 1 bis 5 ist, z 0 oder eine ganze Zahl von 1 bis 2 bedeutet, t 0 oder eine ganze Zahl von 1 bis 3 ist und n 1 bedeutet oder darüber liegt;
worin bedeuten:
- die Gruppen R, R¹¹, R¹² und R¹³ unabhängig voneinander ein Wasserstoffatom; ein Halogenatom; eine geradkettige oder verzweigte Alkylgruppe mit 1 bis 20 Kohlenstoffatomen, vorzugsweise 1 bis 10 Kohlenstoffatomen und besonders bevorzugt 1 bis 6 Kohlenstoffatomen; eine Cycloalkylgruppe mit 3 bis 8 Kohlenstoffatomen; eine Gruppe -C(=Y)R⁵, -C(=Y)NR⁶R⁷ oder -R⁸₃Si; -COCl, -OH, -CN, eine Alkenyl- oder Alkinylgruppe mit 2 bis 20 Kohlenstoffatomen und vorzugsweise 2 bis 6 Kohlenstoffatomen; eine Oxiranylgruppe, eine Glycidylgruppe, eine Alkenyl- oder Alkenylengruppe, die mit einer Oxiranylgruppe oder einer Glycidylgruppe substituiert ist; Aryl, Heterocyclyl, Aralkyl, Aralkenyl; eine Alkylgruppe mit 1 bis 6 Kohlenstoffatomen, in der die Wasserstoffatome ganz oder teilweise entweder durch Halogenatome, wie Fluor, Chlor oder Brom, oder durch eine Alkoxygruppe mit 1 bis 4 Kohlenstoffatomen oder durch eine Arylgruppe, eine Heterocyclylgruppe, -C(=Y)R⁵, -C(=Y)NR⁶R⁷, Oxiranyl oder Glycidyl substituiert sind.
- X ein Halogenatom, wie Cl, Br oder I, oder eine Gruppe -OR', -SR, -SeR, -OC(=O)R', OP(=O)R', -OP(=O)(OR')₂, -OP(=O)OR', -O-NR'₂, -S-C(=S)NR'₂, -CN, -NC, -SCN, -CNS, -OCN, -CNO und -N₃, wobei R' eine Alkylgruppe mit 1 bis 20 Kohlenstoffatomen bedeutet, die ggf. mit einem oder mehreren Halogenen, insbesondere Fluor und/oder Chlor substituiert ist; und R eine geradkettige oder verzweigte Aryl- oder Alkylgruppe mit 1 bis 20 Kohlenstoffatomen und vorzugsweise 1 bis 10 Kohlenstoffatomen bedeutet; wobei die Gruppe -NR'₂ ferner eine cyclische Gruppe bedeuten kann, in der die beiden Gruppen R' so verbunden sind, dass sie einen 5-, 6- oder 7-gliedrigen Heterocyclus bilden können;
- Y O, S oder NR⁸ (vorzugsweise O),
- R⁵ eine geradkettige oder verzweigte Alkyl-, Alkylthio- oder Alkoxygruppe mit 1 bis 20 Kohlenstoffatomen; OH; OM' mit M' = Alkalimetall; eine Aryloxy- oder eine Heterocyclyloxygruppe;
- R⁶ und R⁷ unabhängig voneinander H oder eine geradkettige oder verzweigte Alkylgruppe mit 1 bis 20 Kohlenstoffatomen, wobei R⁶ und R⁷ gemeinsam eine Alkylengruppe mit 2 bis 7 und vorzugsweise 2 bis 5 Kohlenstoffatomen bilden können;
- R⁸ H; eine geradkettige oder verzweigte Alkylgruppe mit 1 bis 20 Kohlenstoffatomen oder eine Arylgruppe.

4. Zusammensetzung nach einem der vorhergehenden Ansprüche, wobei der Initiator ausgewählt ist unter:
- Octa-2-isobutyrylbromid-octa-*t*-butyl-calix(8)aren,
- Octa-2-propionylbromid-octa-*t*-butyl-calix(8)aren, und
- Hexakis-α-bromomethylbenzol.

5. Zusammensetzung nach einem der vorhergehenden Ansprüche, wobei der Ligand ausgewählt ist unter:
(i) Verbindungen der Formel: R⁹-Z-(R¹⁴-Z)ₘ-R¹⁰ oder R²⁰R²¹C[C(=Y)R⁵], worin bedeuten:
- R⁹ und R¹⁰ unabhängig voneinander Wasserstoff; eine geradkettige oder verzweigte Alkylgruppe mit 1 bis 20 und vorzugsweise 1 bis 10 Kohlenstoffatomen; eine Arylgruppe; eine Heterocyclylgruppe; eine Alkylgruppe mit 1 bis 6 Kohlenstoffatomen, die mit einer Alkoxygruppe mit 1 bis 6 Kohlenstoffatomen oder einer Dialkylaminogruppe mit 1 bis 4 Kohlenstoffatomen oder einer Gruppe -C(=Y)R⁵ oder -C(=Y)NR⁶R⁷ und/oder YC(=Y)R⁸ (wobei die Definitionen für R⁵ bis R⁸ und Y in Anspruch 3 angegeben sind) substituiert ist; mit der Maßgabe, dass R⁹ und R¹⁰ gemeinsam einen gesättigten oder ungesättigten Ring bilden können;
- die Gruppen R¹⁴ unabhängig voneinander eine zweiwertige Gruppe, die unter den Alkandiylgruppen mit 2 bis 4 Kohlenstoffatomen; den Alkenylengruppen mit 2 bis 4 Kohlenstoffatomen, die Cycloalkandiylgruppen mit 3 bis 8 Kohlenstoffatomen; den Cycloalkendiylgruppen mit 3 bis 8 Kohlenstoffatomen, den Arendiylgruppen und den Heterocyclylgruppen ausgewählt sind;
- Z O, S, NR¹⁵ oder PR¹⁵ mit R¹⁵=H, eine geradkettige oder verzweigte Alkylgruppe mit 1 bis 20 Kohlenstoffatomen, eine Arylgruppe, eine Heterocyclylgruppe; eine Alkylgruppe mit 1 bis 6 Kohlenstoffatomen, die mit einer Alkoxygruppe mit 1 bis 6 Kohlenstoffatomen oder einer Dialkylaminogruppe mit 1 bis 4 Kohlenstoffatomen oder einer Gruppe -C(=Y)R⁵ oder -C(=Y)NR⁶R⁷ und/oder YC(=Y)R⁸ (wobei die Definitionen für die Gruppen R⁵ bis R⁸ und Y in Anspruch3 angegeben sind) substituiert ist; und
- m im Bereich von 0 bis 6 liegt;
- R²⁰ und R²¹ unabhängig voneinander Wasserstoff; ein Halogenatom; eine geradkettige oder verzweigte Alkylgruppe mit 1 bis 20 und vorzugsweise 1 bis 10 Kohlenstoffatomen; eine Arylgruppe; eine Heterocyclylgruppe; mit der Maßnahme, dass R²⁰ und R²¹ auch gemeinsam einen gesättigten oder ungesättigten Ring bilden können und mit der Maßgabe, dass jede Gruppe ferner mit einer C₁₋₆-Alkylgruppe, einer C₁₋₆-Alkoxygruppe oder einer Arylgruppe substituiert sein kann; und
(ii) Kohlenmonoxid; Porphyrinen und Porphycenen, die ggf. substituiert sind; Ethylendiamin und Propylendiamin, die ggf. substituiert sind; Multiaminen mit tertiären Aminen, wie Pentamethyldiethylentriamin; Aminoalkoholen wie Aminoethanol und Aminopropanol, die ggf. substituiert sind; Glykolen, wie Ethylenglykol oder Propylenglykol, die ggf. substituiert sind; Arenen wie Benzol, die ggf. substituiert sind; Cyclopentadien, das ggf. substituiert ist; Pyridinen und Bipyridinen, die ggf. substituiert sind; Acetonitril; 1,10-Phenanthrolin; Kryptanden und Kronenether; und Spartein.

6. Zusammensetzung nach einem der vorhergehenden Ansprüche, wobei das Treibmittel unter den flüchtigen Kohlenwasserstoffen, wie n-Butan, Propan, Isopropan, Pentan, chlorierten Kohlenwasserstoffen und/oder fluorierten Kohlenwasserstoffen; Kohlendioxid, Distickstoffoxid, Dimethylether (DME), Stickstoff, Druckluft und deren Gemischen ausgewählt ist.

7. Zusammensetzung nach einem der vorhergehenden Ansprüche, wobei das Hilfsmittel zur Filmbildung unter den folgenden Verbindungen oder deren Gemischen ausgewählt ist:
- Glykolen und ihren Derivaten, wie Diethylenglykolethylether, Diethylenglykolmethylether, Diethylenglykolbutylether, Diethylenglykolhexylether, Ethylenglykolethylether, Ethylenglykolbutylether und Ethylenglykolhexylether;
- Glycerinestern wie Glycerindiacetat (Diacetin) und Glycerintriacetat (Triacetin);
- Derivaten von Propylenglykol und insbesondere Propylenglykolphenylether, Propylenglykoldiacetat, Propylenglykolmethylether, Propylenglykolethylether, Propylenglykolbutylether, Dipropylenglykolmethylether, Dipropylenglykolbutylether, Dipropylenglykolethylether, Tripropylenglykolbutylether und Tripropylenglykolmethylether;
- Estern von Säuren und insbesondere Carbonsäuren, beispielsweise Citrate, Phthalate, Adipate, Carbonate, Tartrate, Phosphate und Sebacate,
- ethoxylierten Derivaten, beispielsweise ethoxylierten Ölen und insbesondere pflanzlichen Ölen, wie Ricinusöl; ethoxylierten Siliconölen.

8. Zusammensetzung nach einem der vorhergehenden Ansprüche, wobei das Polymer mit "sternförmiger" Struktur in einer Menge von 1 bis 95 Gew.-% Trockensubstanz, bezogen auf das Gesamtgewicht der Zusammensetzung, vorzugsweise 1 bis 50 Gew.-% und noch bevorzugter 1 bis 20 Gew.-% vorliegt.

9. Zusammensetzung nach einem der vorhergehenden Ansprüche, wobei das Polymer mit "sternförmiger" Struktur in dem Medium in gelöster Form oder in Form einer Dispersion in einer wässrigen, organischen oder wässrig-organischen Phase und insbesondere einer alkoholischen oder wässrig-alkoholischen Phase vorliegt.

10. Zusammensetzung nach einem der vorhergehenden Ansprüche, die als Spray, Lack, Schaum, Creme, Gel, Emulsion, Lotion oder Wachs vorliegt.

11. Verfahren zur Festigung oder Formgebung der Haare, **dadurch gekennzeichnet, dass** auf die Haare eine kosmetische Zusammensetzung nach einem der vorhergehenden Ansprüche aufgebracht wird.

12. Verwendung einer kosmetischen Zusammensetzung nach einem der Ansprüche 1 bis 10 zur Herstellung eines kosmetischen Produkts für das Haar, das dazu vorgesehen ist, die Frisur und/oder die Haare zu festigen und/oder zu fixieren und/oder zu behandeln.

13. Verwendung nach Anspruch 12 zur Herstellung eines Frisierprodukts, beispielsweise eines Lacks, Sprays oder Schaums zur Festigung oder Formgebung der Frisur.
